# EUROPEAN PATENT APPLICATION

(11) **EP 2 923 695 A1**
(43) Date of publication of application: **30.09.2015**
(21) Application number: 14161579.9
(22) Date of filing: 25.03.2014
(51) Int. Cl.: A61K 9/107, C08G 83/00

(54) **Hyperbranched polyglycerol sulfates with hydrophobic cores**

(71) Applicant: DendroPharm GmbH, 14195 Berlin (DE)
(72) Inventor: Paulus, Florian, 12247 Berlin (DE); Haag, Rainer, 12209 Berlin (DE)
(74) Representative: Moré, Solveig Helga

(57) **Abstract**

The present invention relates to the field of drug carrier systems and polymer therapeutics and diagnostics. It provides derivatized unimolecular polyols with a hydrophobic core moiety and a polyanionic, preferably, sulfated hydrophilic shell, in particular to unimolecular hyperbranched sulfated polyglycerol micelles having a hydrophilic shell and a hydophobic core. These may be used for the supramolecular encapsulation and transport of hydrophobic guest molecules into biological systems. The micelles can be applied as a drug carrier system for therapy and for diagnosis. They may also be therapeutic agents on their own. The invention also relates to preparation of said micelles.

## Description

The present invention relates to the field of drug carrier systems and polymer therapeutics and diagnostics. It provides derivatized unimolecular polyols with a hydrophobic core moiety and a polyanionic, preferably, sulfated hydrophilic shell, in particular to unimolecular hyperbranched sulfated polyglycerol micelles having a hydrophilic shell and a hydophobic core. These may be used for the supramolecular encapsulation and transport of hydrophobic guest molecules into biological systems. The micelles can be applied as a drug carrier system for therapy and for diagnosis. They may also be therapeutic agents on their own. The invention also relates to preparation of said micelles.

During the last decades, many efforts have been made to efficiently deliver therapeutic and diagnostic effector molecules to biological systems, and, thereby, e.g., increase the efficacy of a therapy. As effector molecules are often recognized by the immune system and can therefore cause an immune response which inhibits a therapeutic effect, the effector molecules may also need to be protected.

There are two strategies which, optimally, inhibit the recognition of an effector molecule, and efficiently deliver it to the target of interest. On one hand, the effector molecule can be covalently attached to a macromolecule with a certain size. On the other hand, the active agent can be supramolecularly encapsulated and transported, e.g., by amphiphilic molecules which can self-assemble and self-organize in solution. However, self-assembled micelles or liposomal-like structures are very sensitive to dilution.

The lack of micellar stability triggered the invention of so-called unimolecular micelles. A first study utilized a symmetrical, four-directional hydrocarbon cascade polymer with 36 carboxylic acid surface functionalization (Newkome et al., Angew. Chem. Int. Ed. 1991, 9, 1178-1180). The very defined structures were able to unimolecularly host hydrophobic guest molecules Phenol Blue, chlortetracyclin and diphenylhexatriene. Phase-transition anisotropy measurement indicated a location of the guest molecules in the lipophilic interior of the unimolecular micelle. The anionic derivatized surface provided good water-solubility.

Unimolecular, polyanionic micelles were synthesized via a multistep procedure using 3,5-dihydroxybenzyl alcohol building blocks and final modification of the surface functionalities to obtain carboxylates (Hawker et al., J. Chem. Soc., Perkin Trans. 1 1993, 1287-1297). The investigated architectures were able to solvate hydrophobic molecules, e.g., pyrene similar to micelles based on the well-known surfactant sodium dodecyl sulfate (SDS).

With hyperbranched polyglycerol (hPG), a highly biocompatible scaffold with similar properties which can be easily synthesized on a large, semi-industrial scale was introduced (Sunder, et al, Adv. Mater., 2000, 12, 235-239; Frey et al., Rev. Mol. Biotechn., 2002, 90, 257-267; Kainthan et al, Biomacromolecules, 2006, 7, 703-709; Gröger, et al., Bioconjugate Chem., 2013, 24, 1507-1514; Kainthan, et al., Biomaterials, 2007, 28, 4581; Kainthan et al., Biomaterials, 2007, 28, 4779-4787; Imran ul-haq et al., J. Polym. Sci., Part A: Polym. Chem., 2013, 51, 2614-2621). Because of a spherical scaffold, high surface functionality, and a flexible polyether backbone, hPG has become a potent scaffold for various biomedical applications. (Haag et al., Macromolecules, 2000, 33, 8158-8166; Wilms, et al., Acc. Chem. Res., 2009, 43, 129-141; Calderón et al., Adv. Mater., 2010, 22, 190-218).

One strategy to manufacture a unimolecular transporter was based on the mimicry of the liposomal structure with a hydrophilic interior, a hydrophobic bilayer and a hydrophilic shell (Radowski et al., Angew. Chem. Int. Ed. 2007, 46, 1265-1269; Fleige et al. Macromolecules 2012, 45, 9452-9459). The synthesized core-multishell architectures were able to encapsulate hydrophobic and hydrophilic guest molecules in organic and aqueous media. The system was further developed by variation of the outer shell from PEG to polyglycerol dendrons (Fleige et al. Nanocarriers, Vol. 1, 2013, 1-9).

Various scientific investigations described the role of anionic moieties in coagulation, thrombin formation and inflammation (Türk et al. Bioconjugate Chem. 2004, 15, 162-167; Dernedde et al., Proc. Natl. Acad. Sci. U. S. A. 2010, 107, 19679-19684; Weinhart et al. Biomacromolecules 2011, 12, 2502-2511 and Macromol. Biosci. 2011, 11, 1088-1098). In general, anionic moieties inhibit the leukocyte recruitment from the blood stream in inflammatory processes by interaction with the selectin receptors. This prevents the interaction of the L-, P- and E-selectin with the endothelium which is mediated by cell adhesion molecules (Ulbrich et al., Trends Pharmacol. Sci. 2003, 24, 640-647). While WO2011/09531 teaches the preparation of unimolecular polyanionic micelles for the transport of hydrophobic therapeutic and diagnostic effector molecules, these need to be covalently cross-linked to the micelles. This is disadvantageous in some therapeutic approaches, as the effector molecule may stay bound to the micelles.

In view of the state of the art, the inventors solved the problem of providing an improved carrier system for the transport of therapeutic and diagnostic effector molecules. This problem is solved by the invention, in particular, the subject matter of the claims.

The invention provides a unimolecular sulfated polyanionic micelle, in particular, a unimolecular sulfated polyglycerol micelle, having a hydrophilic shell and a hydophobic core. The inventors have shown that, with the combination of a core-shell like architecture and large number of active sulfate targeting moieties, it is possible to target inflamed tissue, mediated the cellular uptake and prevent coagulation without tedious surface modifications and functionalization for covalent effecter molecular attachment.

In the context of the invention, "a" relates to "one or more", unless explicitly mentioned otherwise.

The micelle of the invention preferably has the formula P(OSO₃⁻M⁺)ₙ, wherein P is polymeric polyglycerol wherein a number n of hydroxyl groups is substituted by sulfate groups OSO₃⁻M⁺, wherein M⁺ is a cationic inorganic or organic counter ion to the anionic sulfate group, wherein n preferably is 10 or more. More preferably, n is 30 or more, 60 or more, or 120 or more. Preferably, the degree of sulfation of the hydroxyl groups of the polyglycerol is 30% to 100%, preferably, 60-99%, or 80-95%. "Degree of sulfation" according to this invention means the percentage of sulfated OH groups of the glycerol units of the polymeric polyglycerol core relative to the number of overall OH groups.

The possible number of sulfate groups n depends of the molecular weight of the macromolecule. Based on polyglycerol which comprises repeated units of glycerol units, each unit enables one OH group in the macromolecule. For example, a polyglycerol core of 10 000 g/mol enables 135 OH groups, a polyglycerol core of 2 000 g/mol enables 27 OH groups calculated for a theoretical monodisperse molecule.

Sulfation agents are known in the state of the art, e.g., WO2011/095311. Preferably, a complex of S0₃ and pyridine is used as sulfation reagent. This reagent converts a -OH group into a -OSO3H or - OSO₃⁻-Na⁺-group. Said sulfation reagent is preferably used in a concentration which corresponds to the desired sulfation degree. This means that the sulfation reagent is used in a concentration equimolar or of higher molar equivalents relative to the OH groups of the polymeric polyglycerol core to be converted. The resulting functionalization, i.e. sulfation, can thus be adjusted via the ratio of SO₃ to the OH groups of the polyglycerol.

M⁺ preferably is Na⁺, but it may alternatively be any other single positively charged cationic counter ion M⁺ selected from the group of inorganic alkali metals potassium, lithium, or from organic cationic compounds meglumine, lysine, glycine, or mixtures thereof.

P may be a polymeric polyglycerol comprising repeated units of glycerol with the formula (RO-CH₂)₂CH-OR on a multifunctional starter molecule which is a polyhydroxy compound having 2 to 1,000 hydroxyl groups, wherein R independently is H or a further glycerol unit.

In the micelle of the invention, the core and/or the shell have an average degree of branching of 0 % to 67 %, preferably, 20-67%, 40-67% or 60-67%. "Branching degree" according to this invention means the degree of branching obtained by the reaction of both available OH groups of a glycerol unit with two further monomer molecules during the polymerization process (glycidol in case of the anionic polymerization). A branching degree of 0 describes a fully linear polyglycerol (Gervias, et al., Macromolecules, 2010, 43,01778-1784; Imran ul-haq, et al., Biomaterials, 2012, 33, 9135-9147). Preferably, the polyglycerol is branched. A branching degree of 67% (2/3) is the theoretically achievable maximum for highly branched polyglycerols and means that all OH groups of a glycerol unit have reacted with two further glycerol units. According to the present invention, highly branched polymeric polyglycerol cores with a branching of 20 to 67% are preferably used.

The polymeric polyglycerol core is produced by using a (multi)functional starter molecule or initiator, respectively, during the ring-opening polymerization of glycidol. The starter molecule or initiator, respectively, is a polyhydroxy compound, having 2 to 1,000, preferably 2 to 100 and more preferably 2 to 15, most preferably 2 to 8 OH groups. The starter molecule has the generic formula R-(OH)ₓ, wherein R can be any molecule stable under the conditions of the anionic polymerization, and x is 1 to 1,000; preferably 1 to 100 and more preferably 1 to 15, most preferably 1 to 8. Preferably the used initiators are tris- or tetrafunctional initiators, such as 1,1,1-trishydroxymethylpropane (TMP) or 1,1,1- trishydroxymethylethane (TME), or Benzene-1,3,5-triol (THB) as preferred trisfunctional initiator or pentaerythrol (PE) as preferred tetrafunctional initiator. The starter molecule or the initiator, respectively, can carry further functional groups, such as particularly SH groups, NH₂ groups. In a particular embodiment, the starter molecule contains further heterofunctionalities (like SH, NH₂ derivatized with suited protecting groups). Another starter molecule can be a small polymeric polyglycerol with more than 3, preferably above 10, more preferably above 20 OH groups. Further suitable initiators, heterofunctionalities and protecting groups are known to the person of skill in the art.

The hydrophobic core preferably comprises mono- and/or bi-aromatic polyether moieties, wherein the aromatic moiety may be, e.g., phenyl, functionalized phenyl, naphthyl, functionalized naphthyl, biphenyl, or a functionalized biphenyl derivative. The inventors have shown that it is advantageous for encapsulation of guest molecules if the hydrophobic core comprises a bi-aromatic moiety, e.g., biphenyl. The bi-aromatic moiety may also be naphthyl. Derivatives of biphenyl or naphthyl may also be used.

The hydrophobic core component may have an average molecular weight of 250 to 100 000 g/mol. The hydrophilic shell component may have an average molecular weight of 1000 to 1 000 000 g/mol. The average molecular weight of the micelle may be 1 250 - 5 000 000 g/mol, preferably, 5 000 to 1 000 000 g/mol or 10 000 to 500 000 g/mol. Depending on the choice of the initiator and the polymerization conditions, the polymeric polyglycerol core reaches a branching degree and an arbitrarily adjustable mean molecular weight, which is not a defined molecular weight but a distribution covering a molecular weight range. This so called polydispersity can be described by the polydispersity index (PDI). The PDI is defined as M_{w}/Mₙ, with M_{w} being the weight average molecular mass, and Mn being the number average molecular mass. Preferred polydispersity indices of the employed sulfated polyglycerols are below 3.0, more preferred below 2.5, most preferred below 2.0. The average molecular weights used for the description of the polygylcerol cores are the number average molecular mass Mₙ.

Preferred intermediate products and preferred micelles of the invention are prepared in the examples below and shown in Scheme 1.

Preferably, the micelle of the invention is able to encapsulate and transport a guest molecule, preferably, a hydrophobic therapeutic or diagnostic effector molecule by supramolecular interactions, wherein the guest molecule is not covalently bound. One object of the invention thus is a micelle, as characterized herein, which is supramolecularly encapsulating a guest molecule, preferably, a hydrophobic guest molecule.

The hydrodynamic particle size of the micelle may or may not change upon loading/encapsulation of the guest molecule. For example, the hydrodynamic particle size of the unloaded micelle may be about 130-150 nm, while the hydrodynamic particle size of loaded micelle may be about 130-190 nm.

The guest molecule may be a therapeutic effector molecule, in particular a hydrophobic therapeutic effector molecule, e.g., carbimazol, thiamazol or chlortetracycline. The therapeutic effector molecule may be an anti-cancerogenic, cytostatic, anti-angionetic, anti-inflammatory, anti-bacterial and/or anti-fungal agent, a photosensitizer and/or an siRNA.

The guest molecule may also be a diagnostic effector molecule, e.g., a fluorescent dye such as indocarbocyanine dye (e.g., Cy3), indodicarbocyanine dye, indotricarbocyanine dye, pyrene, fluorescein, rhodamine, perylene dye, croconium dye, squarylium dye, polymethine dye, merocyanine dye, phtalocyanine dye, naphtalocyanine dye, triphenylmethine dye, benzophenoxyazine dye, benzophenothiazine dye, or a chelator with a radioactive or paramagnetic metal.

The inventors have shown that, surprisingly, micelles comprising bi-aromatic moieties in their hydrophobic core were particularly advantageous for encapsulation of hydrophobic guest molecules. They have also shown that, in contrast to micelles generated by statistical copolymerization, micelles with a structured polyglycerol shell prepared by block polymerization were particularly advantageous for encapsulation of guest molecules. The invention thus particularly provides a unimolecular sulfated polyglycerol micelle, having a hydrophilic shell and a hydophobic core comprising bi-aromatic moieties, wherein the polyglycerol structure is obtainable by block polymerization. Said micelle is able to supramulecularly encapsulate a preferably hydrophobic guest molecule.

The present invention also relates to a pharmaceutical composition comprising the micelle of the invention. The pharmaceutical composition may further comprise a biologically acceptable carrier, e.g., water or, preferably, a physiological buffer such as PBS. In a preferred embodiment, the pharmaceutical composition is a lyophilisate, which may further comprise buffer salts, and/or at least one cryoprotectant selected from the group comprising sucrose, mannose and trehalose.

The micelle of the invention may be for use in a method of treating a patient having any a condition or disease selected from the group comprising inflammation, arthritis, otitis media, otitis externa, cancer, autoimmune disease, fibrosis, cartilage defect, osteonecrosis, osteochondritis, cardiovascular disease or sepsis.

The invention also provides the micelle of the invention for use in a method of treating a patient having any disease amenable to therapy by inhibition of NF-kappaB and/or AP-1 and/or TGF-beta synthesis. It has previously been shown that polyanionic hyperbranched polyglycerols, in particular, sulfated hyperbranched polyglycerols, inhibit NF-kappaB, AP-1 and TGF-beta synthesis (WO 2011/095311, Dernedde, et al., Proc. Natl. Acad. Sci. U. S. A., 2010, 107, 19679-19684. Weinhart, et al., Biomacromolecules, 2011, 12, 2502-2511). This also applies for the micelles of the invention.

NF-kappaB, AP-1 and TGF-beta are important therapeutic targets for various diseases and conditions, which are well characterised in the state of the art (Letoha et al, Mol. Pharmacol. 69: 2027, 2006; Sliva et al, Curr Cancer Drug Targets. 4: 327, 2004, WO2011/095311). Treatment of such diseases with the micelles of the invention is specifically envisioned.

WO2011/095311 also demonstrates that sulfated polyols / polyglycerols are specifically targeted to the cytoplasm and nucleus of proliferating and activated cells, i.e., cells having an increased metabolic activity, which may be detectable by the MTT-assay and/or by detection of inflammatory cytokines in the supernatant of activated immune cells, by a mechanism distinct from endocytosis. This also applies to the micelles of the present invention.

The patient may be a human or animal patient, e.g., a cat, dog, horse, pig, cattle, chicken, mouse, rat, rabbit, guinea pig, goat, sheep, camel, fish, monkey or ape.

The micelle is to be administered to the patient. It may be administered once, or in multiple treatments, e.g., with doses of 1 mg/ml to 1 000 mg/kg per administration. Administration may be, e.g., parenteral, e.g., intravenous or subcutaneous, or topical, e.g., dermal.

The invention also relates to a method of treatment of a patient likely to benefit from such treatment comprising administering an effective amount of the micelle of the invention to said patient, wherein the patient preferably has a condition or disease as described above. The disease or condition may also be any disease amenable to therapy by inhibition of NF-kappaB and/or AP-1 and/or TGF-beta synthesis.

The invention also relates to the micelle of the invention for use in a method of diagnosing a disease or condition in a patient, wherein the micelle is to be administered to the patient. The invention also relates to a method of diagnoses of a patient likely to benefit from such treatment comprising administering an effective amount of the micelle of the invention to said patient. For diagnostic applications, the micelle preferably encapsulates a detectable agent, e.g., a fluorescent dye, a radioactive compound or a hapten.

The invention also relates to a method of delivering a guest molecule to a group of cells. The micelle of the invention preferably targets active cells and/or proliferating cells. It has been found that the micelle of the invention may be advantageously used to deliver a guest molecule to bone and/or cartilage, e.g., to osteochondral cells and/or cartilage cells. The micelle supramolecularly encapsulating a guest molecule may be administered to a biological system comprising said cells, e.g., in vitro.

The invention also relates to a method for preparing the micelle of the invention, comprising steps of
a) preparing the micelle's core-shell structure by one or more reaction steps, preferably, in a a one-pot batch reaction applying one-step or two-step procedures, wherein block polymerization is preferably performed, and
b) sulfating the micelles' core-shell structure;
c) optionally, loading a guest molecule into the micelle by solid uptake or by an amorphous solid film.

A set of six hydrophobically derivatized polymers based on polyglycerol sulfates have been investigated by the inventors to determine the influence of scaffold architecture on the encapsulation properties of hydrophobic guests. Each of three block and statistical copolymers has been synthesized with phenyl, naphthyl, and biphenyl substituents in a one-pot procedure. The copolymers have been functionalized with sulfate groups in order to introduce an electrostatically repulsive surface that can stabilize the aggregated carriers. In addition, sulfates provide a highly active targeting moiety for inflammation and cellular uptake. UV measurements show a supramolecular encapsulation of the investigated guest molecules in the low µM range. The transport studies with pyrene and an indocarbocyanine dye further indicated a core-shell-type architecture which provides a distinct amphiphilicity advantageous for supramolecular guest complexation. The combination of a host functionality with an active sulfate targeting moiety may be used for cellular transport and has great potential for inflammation targeting.

As shown in the Examples in detail, the inventors investigated the structure dependent transport properties of aromatically derivatized hyperbranched polyglycerol sulfates (hPGS). Peripheral sulfate groups were introduced due to their outstanding anti-inflammatory potential and as active targeting moieties in biomedical applications. Dernedde, et al., Proc. Natl. Acad. Sci. U. S. A., 2010, 107, 19679-19684. Weinhart, et al., Biomacromolecules, 2011, 12, 2502-2511. Hyperbranched polyglycerol sulfates with a hydrophobic core have been synthesized for the first time. They possess an active targeting moiety, and additional anti-inflammatory potential.

To obtain the hydrophobically derivatized, amphiphilic architectures, three glycidol derivatives, namely, 2-(phenoxymethyl)oxirane (1), 2-(naphthalen-1-yloxymethyl)-oxirane (2) and 2-[(4-phenylphenoxy)methyl]oxirane (3), were copolymerized with glycidol. A set of six scaffolds, applying three block and three statistical copolymerizations, were synthesized. The molar monomer feed ratios were kept constant for the same hydrophobic comonomer to obtain a hydrophobic polymer part with Mₙ = 2,000 g mol⁻¹ and a hydrophilic hPG matrix with Mₙ = 10,000 g mol⁻¹.

All polymerization reactions were performed in a one-pot batch reaction applying one-step or two-step procedures. The influence of aromatic units as well as the position within the polymer scaffold was determined by comparing block and random copolymers. Two block copolymers with bi-aromatic moieties showed transport capacities from < 1 wt%. However, the aggregates with a stable size were formed independent from the presence of a guest molecule. A statistical distribution provided insufficient amphiphilicity to encapsulate any guest molecules although stable aggregates were formed. The uptake of the host-guest complexes and the liberation of the free ICC dye indicated that the combination with an active sulfate targeting moiety renders the carriers into potent drug delivery systems for cellular uptake.

The invention has provided new insight into the prerequisites for potent carriers based on polyglycerol sulfates. The inventors have surprisingly shown that only a core-shell type architecture is efficient for guest transport in contrast to a statistical distribution of the hydrophobic moieties.

The Examples below are provided to further illustrate the invention, and they are not intended to limit the invention's scope. All cited literature is herewith fully incorporated herein.

### Legends

Fig. 1. Hydrodynamic particle size by intensity of sulfated copolymers with 1 mg mL⁻¹ in 150 mM PBS.
Fig. 2. Structures of pyrene (**4**) and ICC dye (**5**) used for encapsulation studies.
Fig. 3. TC of **4** and **5** in sulfated block copolymers **b2S** and **b3S** determined by UV measurement in MeOH based on ε = 45 300 cm⁻¹ M⁻¹ for pyrene and ε = 132 130 cm⁻¹ M⁻¹ for the ICC dye.
Fig. 4: Mean fluorescence at 590 nm (Cy3 dye detection) is displayed for A2780 (4 h: light gray, 24 h: dark gray columns) and A549 (4 h: black, 24 h: white columns) cells incubated with copolymers **b2S** and **b3S,** free ICC dye, and PBS control.
Fig. 5: Confocal laser scanning fluorescence microscopy images from QGP-1 cells after incubation with (a) **b2S** + ICC dye, (b) **b3S** + ICC dye, (c) free ICC dye, and (d) control PBS for 4 h. The merged pictures (a-d) show the ICC dye in red, stained nuclei in blue (DAPI), and the actin cytoskeleton (Alexa Fluor 488 Phalloidin label) in green; (e-h) present ICC dye channel for (e) **b2S** + ICC dye, (f) **b3S** + ICC dye, (g) free ICC dye, and (h) control PBS visualized as intensity grey values.

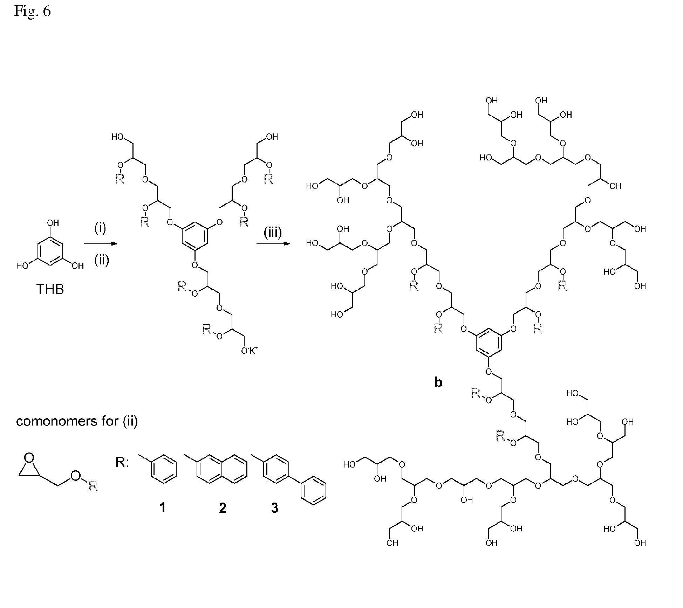

Fig. 6: Scheme 1. Synthesis of core-shell type, block copolymers **b1** to **b3** including steps: (i) KO*t*Bu, NMP, 120 °C, 1 h; (ii) comonomer **1, 2, 3** in THF/NMP, 120 °C, addition over 18 h; (iii) glycidol in THF, 120 °C, addition over 18 h.

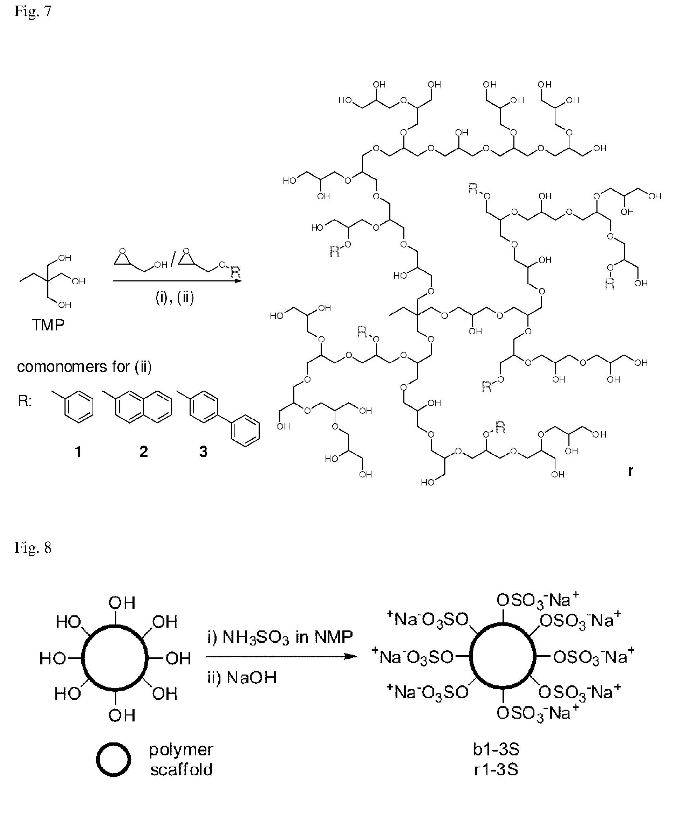

Fig. 7: Scheme 2. General mechanism for the synthesis of statistical, unsulfated copolymers as precursor for PGS **r1** to **r3** including steps: (i) KO*t*Bu, NMP, 120 °C, 1 h, and (ii) glycidol/comonomer **1, 2, 3** mixture in THF/NMP, 120 °C, addition over 20 h. The scheme presents the synthesis of the block copolymer precursor for PGS **r.1.**
Fig. 8: Scheme 3. Sulfation of hydroxyl terminated polymer architectures: (i) NH₃SO₃, NMP, 90 °C, 3 d; (ii) neutralization with NaOH to pH 10-12.

### Examples

### Materials

All chemicals were purchased from Acros Organics and used as received if not otherwise indicated. Glycidol was dried over CaH₂ and distilled under reduced pressure. The purified monomer was stored at 4 °C under inert atmosphere and only used up to two months. All solvents were purchased from Sigma-Aldrich and used without further purification. Dry tetrahydrofuran (THF) was obtained from a solvent purification system. Dry N-methyl-2-pyrrolidone (NMP) and potassium tert-butoxide (KOtBu) were used and stored under AcroSeal® conditions and used as received. Monomers **2** and **3** were synthesized according to published procedures. Indocarbocyanine dye (ICC) was obtained from mivenion GmbH.

### Block copolymerization

The batch reactor was extensively dried at 140 °C under reduced pressure. After cooling to 65 °C, 1,3,5-Trihydroxybenzene (THB), KO*t*Bu, and dry NMP were added under inert conditions. The temperature was increased to 120 °C whereas butanol was distilled and the initiator homogeneously dissolved. The glycidol derivative **1, 2** or **3** (see Table 1) was mixed with THF/NMP, and slowly added to the reactor over a period of 18 h using a precision dosing pump. Subsequently, a solution of glycidol in THF was added to the reactor over 18 h and additionally stirred for another 2 h. After cooling to ambient temperature, the polymer mixture was diluted with methanol and cation exchange resin (Dowex® Monosphere® 650C UPW, Supelco, Belefonte, USA) was added. After stirring for 3 d, the turbid solution was filtered and remaining solvent was evaporated under reduced pressure. Purification of a partial batch amount was performed by dialysis in saturated aqueous NaCl solution. ¹H NMR spectra are available in Figure S 1.

*PPhGE-block-hPG:* M_{w} = 4.3 kDa, PDI = 2.78; M_{w,Aggregate} = 126 kDa, PDI = 1.91; ¹H NMR (700 MHz, DMSO-*d*₆, δ): 7.5 - 6.5 (m, 5H, Ar H), 4.9 - 4.2 (m, 1H, OH), 4.0 - 3.0 (m, 5H, hPG backbone).

*PNpGE-block-hPG:* M_{w} = 1.6 kDa, PDI = 3.55; M_{w,Aggregate} = 130 kDa, PDI = 1.75; ¹H NMR (700 MHz, DMSO-*d*₆, δ): 8.5 - 7.2 (m, 7H, Ar H), 4.9 - 4.2 (m, 1H, OH), 4.2 - 3.0 (m, 5H, hPG backbone).

*PbPhGE-block-hPG:* M_{w} = 2.4 kDa, PDI = 1.23; M_{w,Aggregate} = 105 kDa, PDI = 1.81; ¹H NMR (700 MHz, DMSO-*d*₆, δ): 7.7 - 6.8 (m, 9H, Ar H), 5.0 - 4.2 (m, 1H, OH), 4.2 - 3.0 (m, 5H, hPG backbone).

### Statistical copolymerization

The batch reactor was extensively dried as described. Trimethylolpropane (TMP) was melted at 65 °C under reduced pressure. KOtBu and NMP were added under inert conditions and the temperature was increased to 120 °C. A defined monomer mixture of glycidol with **1, 2** or **3** (see Table 1) in THF was prepared and slowly added to the reactor over a period of 20 h whereas the THF was immediately distilled from the reaction mixture. After complete monomer addition and stirring for additional 2 h, the polymer mixture was diluted with methanol and cation exchange resin was added. After stirring for 3 d, the polymer solution was filtered and solvent was evaporated under reduced pressure. Purification of a partial batch amount was performed by dialysis in water for 3 d. ¹H-NMR spectra are available in Figure S 1.

*PPhGE-co-hPG:* M_{w} = 5.1 kDa, PDI = 1.75; ¹H NMR (700 MHz, DMSO-*d*₆, δ): 7.4 - 6.8 (m, 5H, Ar H), 5.1 - 4.2 (m, 1H, OH), 4.2 - 3.0 (m, 5H, hPG backbone).

*PNpGE-co-hPG:* M_{w} = 5.6 kDa, PDI = 1.83; ¹H NMR (700 MHz, DMSO-*d*₆, δ): 8.5 - 6.8 (m, 7H, Ar H), 5.2 - 4.2 (m, 1H, OH), 4.2 - 3.0 (m, 5H, hPG backbone).

*PbPhGE-co-hPG:* M_{w} = 5.8 kDa, PDI = 1.87; ¹H NMR (700 MHz, DMSO-*d*₆, δ): 7.7 - 6.8 (m, 9H, Ar H), 5.0 - 4.2 (m, 1H, OH), 4.2 - 3.0 (m, 5H, hPG backbone).

| Table 1.Polymerization of hydrophobically derivatized block and statistical composite copolymers including monomer feed ratios and characterization. | | | | | | | | | | |
|---|---|---|---|---|---|---|---|---|---|---|
| | | | | | | | | | | |

| Entry | Initiator [mmol] | Comonomer [mmol] | Glyicdol [mmol] | I*_{r,target}* [%] | Mₙ [kDa] (PDI) | M_{n,Aggr.} [kDa] (PDI)^{a} | I*_{r,measured}* [%]^{b} | DS [%]^{c} | d [nm] ± *SD*^{d} | Copolymer |
|---|---|---|---|---|---|---|---|---|---|---|
| b1 | 5.00 | 66.6 | 718 | 9.3 | 4.3 (2.78) | 126 (1.91) | 2.7 | 99 | 88 ± 3 | PPhGE-block-hPGS **(b1S)** |
| b2 | 2.13 | 21.2 | 287 | 7.4 | 1.6 (3.55) | 130 (1.75) | 5.3 | 84 | 143 ± 8 (21 ± 1) | PNpGE-block-hPGS (**b2S**) |
| b3 | 3.93 | 35.4 | 574 | 6.2 | 2.4 (1.23) | 105 (1.81) | 6.2 | 64 | 136 ± 5 (18 ± 1) | PbPhGE-block-hPGS (**b3S**) |
| r1 | 0.97 | 13.4 | 144 | 9.3 | 5.1 (1.75) | n.d. | 6.7 | 96 | 222 ± 10 (8 ± 1) | PPhGE-co-hPGS **(r1S)** |
| r2 | 0.95 | 10.6 | 143 | 7.4 | 5.6 (1.83) | n.d. | 6.2 | 91 | 222 ± 11 (10 ± 1) | PNpGE-co-hPGS (**r2S**) |
| r3 | 0.94 | 8.8 | 143 | 6.2 | 5.0 (1.87) | n.d. | 5.7 | 93 | 237 ± 8 (21 ± 2) | PbPhGE-co-hPGS **(r3S)** |
| ^{a}Molecular weight of amphiphilic core structure determined by GPC in organic solvent NMP relative to PEG standard. "n.d." indicates "not detectable". ^{b}Comonomer incorporation was determined from ¹H NMR integrals. ^{c}DS was determined by elementary analysis. ^{d}Hydrodynamic diameters of the aggregated copolymers measured after intensity; small particles included as secondary species in brackets (occurrence < 2.5%). | | | | | | | | | | |

### Sulfation of polymer architectures

Sulfation of the polymer architectures was performed as follows: 120 mg of each copolymer was dissolved in NMP and 1.5 to 1.8 equivalents NH₃SO₃ per hydroxyl group were added. The samples were sonicated for 10 min and subsequently stirred at 90 °C for 3 d. After cooling to room temperature the polymer mixture was precipitated into diethyl ether and stirred for 30 min. The solvent was decanted and purification was performed by dialysis in saturated aqueous NaCl solution for 3 d with solvent exchange twice a day. ¹H NMR spectra are available in Figure S2. The degree of sulfation (DS) was determined by combustion analysis (see Table 1 and Table S1).

### Encapsulation of guest molecules

A stock solution of the guest molecules (**4** and **5**) with 5 mg mL⁻¹ in THF or dichloromethane (DCM) was prepared. 200 µL of the stock solution was transferred into a small vial and the solvent was evaporated. In the meantime, a stock solution of each synthesized copolymer with 1 mg mL⁻¹ in in Dulbecco's phosphate buffered saline (DPBS, 1x, Sigma-Aldrich, Germany) was prepared. After evaporation of the solvent, 3 mL of each polymer solution and 3 mL of pure PBS was added to the dried dyes. After excessive stirring for 24 h, the samples were filtered through 0.45 µm Minisart RC 15 syringe filters (Sartorius Stedim Biotech, Germany).

### Instrumentation

NMR spectra were recorded on a Joel ECX 400 MHz or a Bruker Avance 700 MHz spectrometer as indicated. Spectra were recorded in ppm and referenced to indicated, deuterated solvents. Molecular weight distributions were determined by means of GPC coupled to a refractive-index detector (RI) obtaining the complete distribution (Mₙ, Mp, M_{w}, dispersity). The unsulfated copolymers were measured in NMP with 0.05M LiBr (1.5 mg mL⁻¹) as mobile phase with a flow rate of 0.8 mL min⁻¹ on a GPC (Thermo Separation Products, Thermo Scientific, Waltham, USA) consisting of a solvent delivery system with pump P-100 and an auto sampler AS-100. Two 30 cm columns (PPS: Polymer Standards Service GmbH, Germany; Gram 100Å, 1000Å with 7 µm particle size) were used to separate the samples. The columns were operated in a column oven at 70 °C. The calibration was performed by using linear calibration standard (PSS GmbH, Germany). Aqueous samples (sulfated architectures) were measured under highly diluted conditions (10 mg mL¹) from a GPC consisting of an Agilent 1100 solvent delivery system with pump, manual injector, and an Agilent differential refractometer. Three 30 cm columns (PPS GmbH, Germany; Suprema 100Å, 1000Å, 3000Å with 5 and 10 µm particle size) were used to separate aqueous polymer samples using water with 0.05% NaNO₃ as the mobile phase at a flow rate of 1 mL·min-1. The columns were operated at ambient temperature with the RI detector at 50 °C. The calibration was performed by using linear pullulan calibration standard (PPS GmbH, Germany). WinGPC Unity from PSS was used for data acquirement and interpretation. Particle size was determined by DLS in UV-transparent low-volume disposable polystyrene cuvettes on a Zetasizer Nano ZS (Malvern Instruments Ltd., UK) equipped with a 4 mW He-Ne laser (λ = 633 nm). The samples were measured at a concentration of 1 mg mL⁻¹ in DPBS at pH 7.4. Prior to the measurement all samples were filtered through 0.45 µm Minisart RC 15 syringe filters. Each sample was equilibrated for 1 min at 25 °C and measured with 10 scans for 15 s (173° back-scattering). All stated values are the mean of at least 3 independent measurements and standard deviation is indicated with ± *SD.* UV/Vis spectra were recorded on a SCINCO S-3100 UV/Vis spectrometer equipped using UV Disposable Cuvettes 220 nm - 900 nm (BrandTech Scientific, Inc., Essex, USA). The transport capacities were determined as follows: 1 mL of aqueous polymer solution in PBS was freeze dried. The dried polymer-dye-complex was suspended in MeOH to extract the dye from the polymer scaffold and extensively stirred for 18 h. Subsequently the suspensions were filtered through 0.45 µm Minisart RC 15 syringe filters (Sartorius Stedim Biotech, Germany).

### Cell preparation for fluorescence microscopy and FACS analysis

For confocal laser scanning microscopy, the epithelial human pancreatic cancer cell line QGP-1, the human lung carcinoma epithelial cell line A549 and the ovarian carcinoma cell line A2780 were routinely propagated as follows: DEMEM medium, with 10% fetal calf serum (FCS), 2% glutamine, and penicillin/streptomycin (all from PAN Biotech) added. Cells were seeded into medium at 1x10⁵ cells mL⁻¹, cultured at 37 °C with 5% CO₂, and split 1:5 two times a week. For cytochemistry, cells were seeded at 5x10⁴ cells mL⁻¹ in a 24-well culture plate on glass coverslips (Sigma), and cultured at 37 °C for 24 h. Thereafter, cells were incubated with normal culture medium or medium containing 10⁻⁶ M of dye-labeled test substances for QGP-1 cells and 5.0 x 10⁻⁷ M of dye labeled test substances for A2780 and A549 cells at 37 °C for 4 or 24 h. Afterwards, cells were fixed with cold acetone, rinsed and covered with Alexa Fluor 488 Phalloidin (1:300, Molecular Probes) only for QGP-1 cells. 4,6-diamidino-2-phenylindole (DAPI, Abcam) was used for nuclear counterstain. Confocal images were acquired at room temperature with a 63x 1.4 NA HC PL APO CS2 oil objective suited in a confocal microscope (TCS-SP8, DMI6000 stand; Leica). Images of different groups were acquired with the same laser and detector settings using the Leica LAS AF software. The fluorescence detection was performed sequentially for each channel set with the acousto optical beam splitter between 500 and 530 nm for the Alexa Fluor 488 and between 590 and 630 nm for the ICC dye. Alexa Fluor 488 was excited using the 488-nm Argon laser line, whereas the ICC dye was excited with a 561-nm diode-pumped solid-state laser. DAPI was excited with an UV laser (405 nm) and fluorescence detection was set with the acousto optical beam splitter between and 420 and 470 nm. For FACS analysis, the three different cell lines were routinely propagated as described. The cells were cultured 4x10⁴ cells mL⁻¹ cells were cultured in 48-well-plates at 37 °C for 24 h. Thereafter, cells were incubated with normal culture medium or medium containing 10⁻⁶ M test substance for 4 or 24 h. Thereafter, cells were washed with PBS, detached with 200 µL per well accutase (PAA), and centrifuged with 350 g, at 4 °C for 5 min. Supernatants were removed and cells were suspended in 50 µL PBS with 0.5% bovine serum albumin (Roth). Cells were immediately analyzed in a FACS Calibur analysis instrument (Becton-Dickinson: FL2 BP 585/42 nm).

### Results and discussion

### Synthesis

The monomer to initiator ratio for all synthesized copolymers was adjusted to yield a hydrophilic PG matrix of Mₙ = 10,000 g mol⁻¹ with a hydrophobic contribution of Mₙ = 2,000 g mol⁻¹ to yield an overall targeted molecular weight of 12,000 g mol⁻¹. 1,3,5-Trihydroxybenzene (THB) was applied as an initiator for the block copolymerization to obtain a star-like hydrophobic core with an additional central benzene moiety (Scheme 1). Trimethylolpropane (TMP) was used as an initiator for the statistical copolymerization (Scheme 2).

### Synthesis of block copolymers

The amphiphilic block copolymers were successfully synthesized by a one-pot, two-step procedure (see Scheme 1). After the synthesis of the hydrophobic core structure, a PG shell was added to provide sufficient solubility in aqueous media. With the presented strategy, it was possible to obtain more defined core shell structures as the monomers were added stepwise.

In comparison to post-synthetic modification protocols, where secondary hydroxyl groups were functionalized all over the scaffold, the hydrophobic moieties were exclusively located in the core in this new approach.

The amphiphilic structures formed large aggregates in buffered solution. Although the hydrophilic outer shell had about 5-fold the molecular mass of the hydrophobic core, it was not enough to prevent aggregation (see Table 2). Therefore, a unimolecular transport system could not be expected. The main species present in the particle size distribution ranged between 113 and 160 nm for the OH-terminated copolymers 1.1 to 1.3. The aggregation was not only present in DLS but also visible in the GPC measurements (see Table 1). Even the highly polar, organic solvent NMP could not prevent aggregation. Large aggregates with Mₙ > 100 kDa were detected by the RI-detector referenced to PEG, in all block copolymers.

The amount of incorporated comonomer into the hydrophobic core determined by NMR was below the targeted content for **b1** and **b2.** Although the monomer was added slowly to the initiator, small amounts were consumed by side reactions, e.g., self-initiation, and therefore not incorporated into the core structure. The reduced values, however, can also be explained by a strong shielding of the inner core protons as well as reduced signals from aggregation. Due to heavy aggregation at higher concentrations, the NMR characterization was performed under high dilution. From experiences in NMR characterization of polymers, the concentration was too low for an adequate NMR concentration.

### Synthesis of statistical composite polymers

The statistical composite polymers were synthesized by simply feeding the batch reactor with a premixed monomer mixture of glycidol with monomer **1, 2,** and **3.** The same comonomer to glycidol ratio was applied as for the block copolymers **b1** to **b3** (Scheme 2). Trimethylolpropane (TMP) was used as initiator.

The monomer feed was slowly added to the reaction vessel and copolymers **r1** to **r3** were obtained as brownish, turbid wax after work up. The molecular weights determined by GPC in NMP were in the same range but below the targeted molecular weights. No aggregate peaks were detected in the GPC measurement for the statistical copolymers, but particle sizes were determined by DLS to be between 235 and 409 nm. These sized aggregates are usually filtered on the pre-column of the GPC equipment.

The hydrophobic comonomers were incorporated into the PG matrix similarly to the block copolymers but were again below the targeted values, especially for **r1.** Assuming comparable reactivity to the other monomers and a homogeneous incorporation into the scaffold, the resonance signal was probably reduced by shielding and aggregation phenomena similar to the block copolymers. This effect was more pronounced because of the aggregate size determined by DLS.

### Polyglycerol sulfate derivatives with hydrophobic cores

All the investigated copolymer species were sulfated to introduce an electrostatic repulsion to the polymer surface and include a targeting function. Preliminary encapsulation experiments with pyrene otherwise indicated heavy aggregation during loading, which caused the complexes to precipitate even at low concentrations. Therefore, all copolymer species were sulfated using an excess of sulfamic acid (NH₃SO₃) in NMP as seen in Scheme 3.

The sulfation was performed in NMP using NH₃SO₃. The lower reactivity than in other SO₃ complexes was compensated by increasing both reaction temperature and time. The degrees of sulfation (DS) are presented in Table 1. Although the sulfation of all copolymers was conducted according to the same protocol, the DS for polymers **b2S** and **b3S** decreased. This reduction may be a result from aggregation during the reaction. Although all samples were sonicated prior to heating and extensively stirred throughout the reaction, aggregation could not be prevented during functionalization. Therefore, a certain number of hydroxyl groups were not sulfated. Its amphiphilic character and the accessibility of the sulfate groups is in line with the discussed assumption.

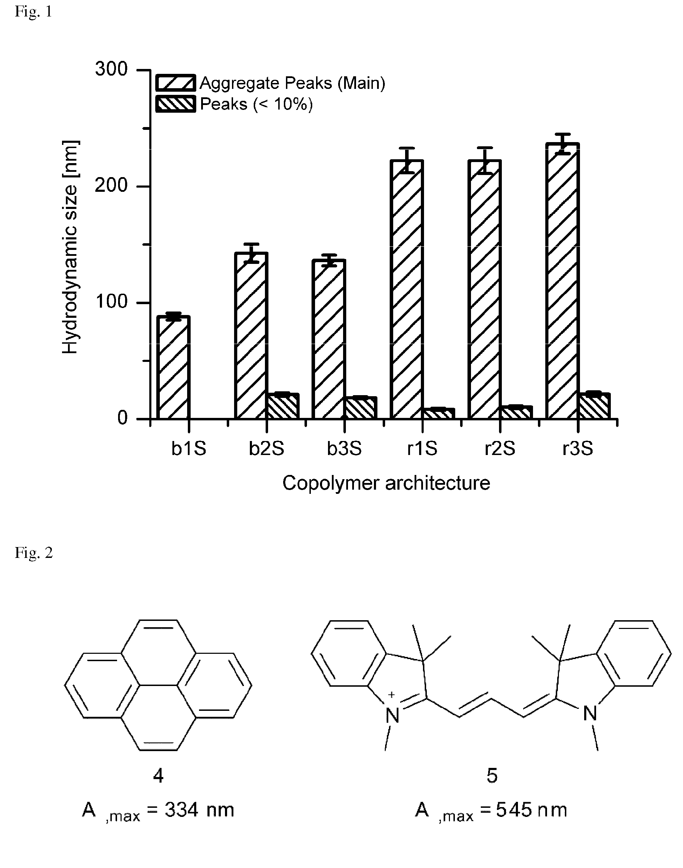

The sulfated copolymers were investigated by DLS to determine the particle sizes under buffered conditions prior to dye encapsulation (see Figure 1). All architectures except **b1S** showed two distinct particle sizes. However, the occurrence of the peaks indicated that the polymers were mainly aggregated. The PGS copolymers showed similar hydrodynamic sizes depending on the nature of the architecture, which were either block or statistical copolymers. The block copolymers were around 139 ± 3 nm and the statistical around 227 ± 7 nm in size.

Copolymer **b1S** had a significant deviation from the related structures which can be explained by an insufficient amphiphilicity.

### Encapsulation of guest molecules

The encapsulation properties of the sulfated copolymers were investigated using pyrene (4) and indocarbocyanine dye (5, ICC, Cy3) as presented in Figure 2. Both dyes were encapsulated using the previously described film method. (Kurniasih et al., New J. Chem., 2012, 36, 371-379; Fleige, et al., in Nanocarriers, 2013, vol. 1, pp. 1-9).

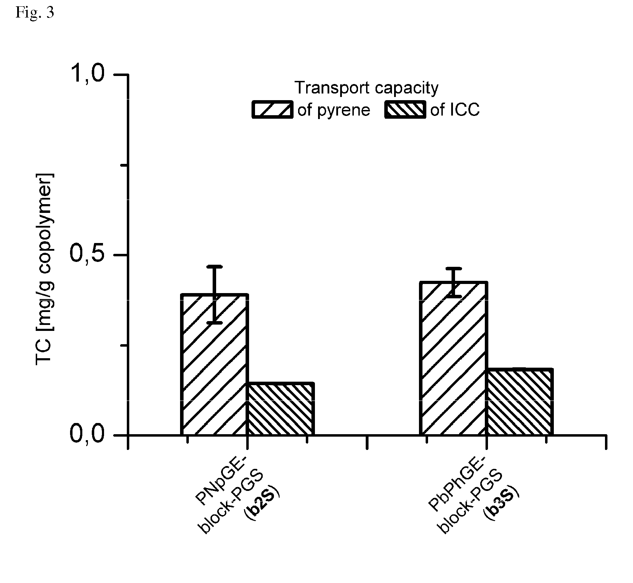

The ICC dye (5) was investigated due to an applicable emission pattern with λ > 600 nm, which has been already exploited in several cellular uptake studies with ICC labeled polyglycerol sulfates due to its tissue permeating properties. In the previously conducted assays, however, the ICC was covalently attached to the PGS scaffold which included numerous functionalization steps. The inventors wanted to investigate the potential use in therapeutic applications with the supramolecular carriers of the invention. Two of the investigated architectures showed transport capacities (TC) in the low µM range. Figure 3 presents the TC for **b2S** and **b3S** measured by UV absorbance based on extinction coefficients of ε = 45 300 cm⁻¹ M⁻¹for **4** and ε = 132 130 cm⁻¹ M⁻¹ for **5.**

Both TCs of **b3S** with 0.424 and 0.182 mg g⁻¹ copolymer, and of **b2S** with 0.390 and 0.144 mg g⁻¹ **(4** and **5),** are lower compared to previously investigated architectures, but high enough for the cellular uptake studies. Kurniasih et al. Macromol. Rapid Commun., 2010, 31, 1516-1520; Fleige, et al., in Nanocarriers, 2013, vol. 1, pp. 1-9; Steinhilber et al., MRS Proceedings, 2012, 1403). Regarding the hydrodynamic sizes before and after dye encapsulation, both conjugates showed completely different loading structure relationships (see Table 2). The biphenyl derivatized structure **b3S** had similar particle sizes to those in the unloaded architecture. The amphiphilicity induced by hydrophobic core seems to successfully stabilize the supramolecular aggregates.

The naphthyl derivatized architecture showed a similar particle size to the unloaded structure upon loading with pyrene, but the hydrodynamic diameter increased by roughly 25% upon loading with ICC dye. In this case, neither the electrostatic repulsion nor the amphiphilic core shell structure could stabilize the aggregates. In both polymer dye complexes, the surface charge prevented precipitation. Although the degree of sulfation (DS) decreased for **b2S** and **b3S,** the lower DS combined with sufficient amphiphilic properties was able to stabilize the aggregates without a guest molecule. The achieved loading is mainly affected by the overall low hydrophobic content within the polymer architecture (1:5). A distinct structure activity relationship was determined, as both block copolymers which contain a bi-aromatic moiety **b2S** and **b3S** were able to carry a guest molecule.

No dye encapsulation was detected for the copolymers **r1S** to **r3S.** Although the same comonomer incorporation as for the block copolymers was achieved, the statistical distribution over the whole scaffold obviously reduced the hydrophobicity below the required threshold. From a previous study, the inventors were aware that hydrophobic core functionality is important for encapsulation properties and can influence the maximum loading capacity. Here, a higher core functionalization, similar to the synthesized random composite polymers **r1S** to **r3S,** resulted in a decrease of loaded guest molecule, whereas lower functionalization showed higher capacities. In general, the hydrophobic ratio is crucial for the encapsulation of guest molecules as a distinct amphiphilicity has to be introduced to the PG scaffold.

| Table 2. Hydrodynamic particle sizes of unloaded and loaded core shell architectures. | | | | | |
|---|---|---|---|---|---|
| | | | | | |

| Carrier | d ± *SD^{a}* [nm] | Pyrene (4) | | ICC (5) | |
|---|---|---|---|---|---|
| | | TC [mg/g] | d_{loaded} [nm] | TC [mg/g] | d_{loaded} [nm] |
| b2S | 143 ± 8 (21 ± 1) | 0.390 | 133 ± 3 (22 ± 2) | 0.144 | 187 ± 6 |
| b3S | 136 ± 5 (18 ± 1) | 0.424 | 144 ± 3 (24 ± 2) | 0.182 | 131 ± 3 (23 ± 2) |
| ^{a}Hydrodynamic diameters of the aggregated copolymers measured after intensity; small particles included as secondary species in brackets (occurrence < 2.5%). Standard deviations are included with ± *SD.* | | | | | |

### Cellular uptake of host/guest complexes

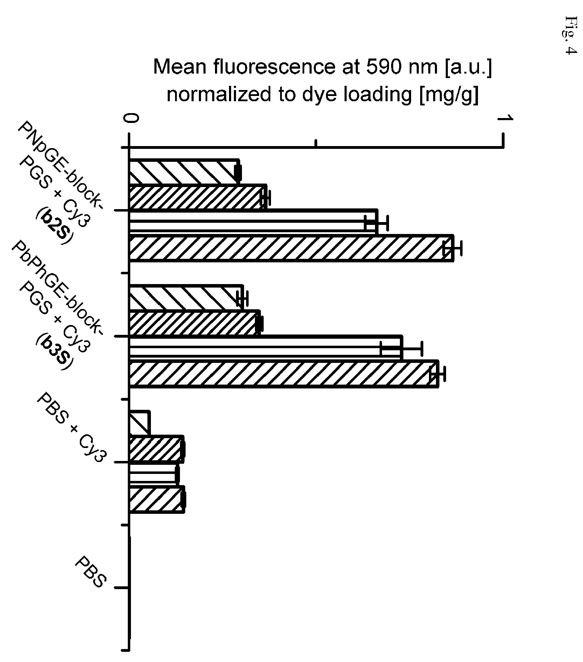

The dye loaded block copolymers **b2S** and **b3S** were investigated for their cellular uptake properties into the QGP-1 pancreatic carcinoma cell line, A549 human lung carcinoma epithelial cell line and A2780 ovarian carcinoma cell line. Previous studies showed an enhanced cellular uptake of dye labeled dPGS (Licha et al., Bioconjugate Chem., 2011, 22, 2453-2460; Biffi et al., PLoS One, 2013, 8, 1-9). The cellular uptake was compared after 4 h and 24 h by utilizing fluorescence activated cell sorting (FACS) according to a standardized protocol (Reichert et al., Small, 2011, 7, 820-829). The uptake into highly proliferating A2780 and A549 cells increased with incubation time and was similar for the cross-comparison of the normalized values for both nanocarriers **b2S** and **b3S** (see Figure 4). The amount of dye which is endocytosed into the cell is approximately 6-fold for A2780 cells after 4 h and 3-fold after 24 h compared to the free dye. The cellular uptake into A549 is even more pronounced and about 6- to 7-fold after 4 and 24 h, respectively.

The results obtained by FACS analysis show that the supramolecular PGS-dye complexes **b2S** and **b3S** are endocytosed significantly better than the free dye. This is a first indication for the transport potential of the synthesized carriers.

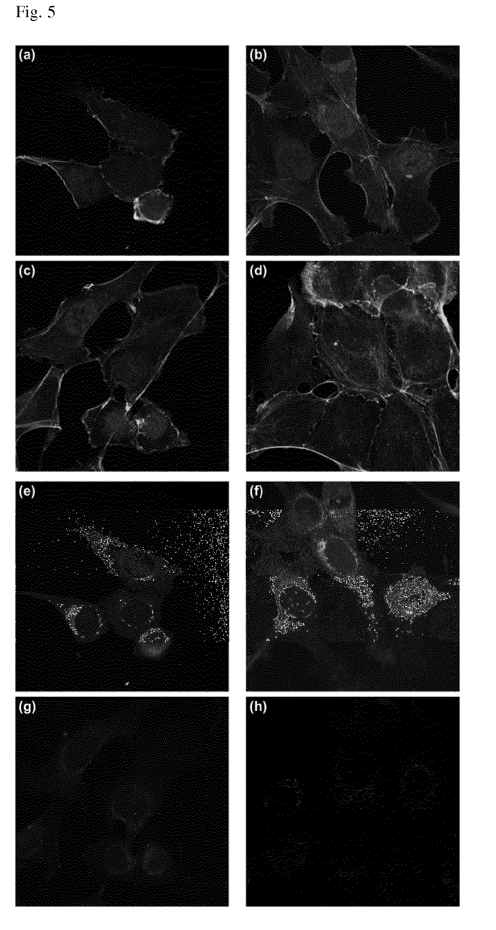

Further characterization of the cellular uptake and transport properties were performed by confocal laser scanning microscopy (CFM) after incubation times of 4 and 24 h into all three cell lines. The merged pictures from the uptake into QGP-1 cells (Figure 5) indicated an increased and controlled uptake into the perinuclear region of the living cells after 4 h. In comparison, the free dye did not endocytose into QGP-1 cells. CFM pictures from the uptake into A2780 cells showed a controlled uptake of the polymer-dye complex. Compared to the free ICC dye which is randomly distributed over the whole cell nuclei, the complex is accumulated in the perinuclear region. The CFM pictures of the A549 cells present a similar uptake and transport behaviour. However, there was hardly any uptake of the free dye visible. The present study confirmed the uptake of PGS into the perinuclear region of the cell. In the previous studies, however, the ICC dye was covalently attached to the PG scaffold and therefore could not be liberated.

## Claims

1. A unimolecular sulfated polyglycerol micelle having a hydrophilic shell and a hydophobic core.

2. The micelle of claim 1 having the formula P(OS0₃⁻M⁺)ₙ, wherein P is polymeric polyglycerol wherein a number n of hydroxyl groups is substituted by sulfate groups OSO₃⁻M⁺, wherein M⁺ is a cationic inorganic or organic counter ion to the anionic sulfate group, wherein n preferably is 10 or more.

3. The micelle of claim 2, wherein P is a polymeric polyglycerol comprising repeated units of glycerol with the formula (RO-CH₂)₂CH-OR on a multifunctional starter molecule which is a polyhydroxy compound having 1 to 1,000 hydroxyl groups, wherein R independently is H or a further glycerol unit.

4. The micelle of any of the preceding claims, wherein the degree of sulfation of the hydroxyl groups of the polyglycerol is 30% to 100%, preferably, 60-99%.

5. The micelle of any of the preceding claims, wherein the hydrophobic core comprises mono- and/or bi-aromatic polyether moieties, wherein the aromatic moiety is selected from the group comprising phenyl, functionalized phenyl, naphthyl, functionalized naphthyl, biphenyl, and functionalized biphenyl derivatives.

6. The micelle of any of the preceding claims, wherein the hydrophobic core comprises bi-aromatic moiety, preferably, biphenyl, naphthyl or derivatives thereof.

7. The micelle of any of the preceding claims, wherein the hydrophobic core component has a molecular weight of 250 to 100 000 g/mol, and/or wherein the hydrophilic shell component has a molecular weight of 1000 to 1 000 000 g/mol.

8. The micelle of any of the preceding claims, wherein the core and/or the shell has an average degree of branching of 0 to 67 %, wherein the micelle preferably comprises a highly branched sulfated polyglycerol.

9. The micelle of any of the preceding claims, supramolecularly encapsulating a guest molecule, preferably, a hydrophobic guest molecule.

10. The micelle of claim 9, wherein the guest molecule is a therapeutic effector molecule or a diagnostic effector molecule.

11. A pharmaceutical composition comprising the micelle of any of the preceding claims, and, optionally, a biologically acceptable carrier.

12. The micelle of any of claims 1-10 for use in a method of treating a patient having a condition or disease selected from the group comprising inflammation, arthritis, otitis media, otitis externa, cancer, autoimmune disease, fibrosis, cartilage defect, osteonecrosis, osteochondritis, cardiovascular disease or sepsis, any disease amenable to therapy by inhibition of NG-kappaB and/or AP-1 and/or TGF-beta synthesis, wherein the micelle is to be administered to the patient.

13. The micelle of any of claims 1-10 for use in a method of diagnosing a disease or condition in a patient, wherein the micelle is to be administered to the patient.

14. A method of delivering a guest molecule to a group of cells selected from the groups of active cells, proliferating cells, osteochondral cells and cartilage cells, comprising administering the micelle of any claims 9-10 or the pharmaceutical composition of claim 11, wherein the micelle supramolecularly encapsulates a guest molecule, to a biological system comprising said cells, e.g., in vitro.

15. A method for preparing the micelle of any of claims 1-10, comprising steps of
a) preparing the micelle's core-shell structure, and
b) sulfating the micelle;
c) optionally, loading a guest molecule into the micelle by solid uptake or by an amorphous solid film.
